# EUROPÄISCHE PATENTSCHRIFT

(11) **EP 0 582 079 B1**
(45) Veröffentlichungstag und Bekanntmachung des Hinweises auf die Patenterteilung: **12.03.1997**
(21) Anmeldenummer: 93110227.1
(22) Anmeldetag: 26.06.1993
(51) Int. Cl.: C09B 1/22, C09B 1/24

(54) **Verfahren zur Herstellung von 1,4-Diaminoanthrachinon-2,3-disulfonsäure und 1,4-Diaminoanthrachinon-2,3-dinitril**
Method to produce 1,4-diaminoanthraquinone-2,3-disulphonic acid and 1,4-diaminoanthraquinone-2,3-dinitrile
Procédé de préparation d'acide-1,4-diaminoanthraquinone-2,3-disulfurique et 1,4-diaminoanthraquinone-2,3-dinitrile

(30) Priorität: 08.07.1992 DE 4222302
(43) Veröffentlichungstag der Anmeldung: 09.02.1994
(73) Patentinhaber: BASF Aktiengesellschaft, 67063 Ludwigshafen (DE)
(72) Erfinder: Dust, Matthias, Dr., D-6700 Ludwigshafen (DE); Bergmann, Udo, Dr., D-6140 Bensheim (DE); Schwantje, Gerd, Dr., D-6706 Wachenheim (DE)

(56) Entgegenhaltungen:
- GB-A- 2 034 737
- CHEMICAL ABSTRACTS, vol. 100, no. 16, April 1984, Columbus, Ohio, US; abstract no. 122764f, SUMITOMO CHEM. CO. LTD. '1,4-diaminoanthraquinone-2,3-disulfonic acid.' Seite 82 ;Spalte 2 ;
- PATENT ABSTRACTS OF JAPAN vol. 6, no. 266 (C-142)(1144) 25. Dezember 1982 & JP-A-57 158 261 (SUMITOMO KAGAKU KOGYO K. K.) 30. September 1982

## Beschreibung

Die vorliegende Erfindung betrifft ein verbessertes Verfahren zur Herstellung von 1,4-Diaminoanthrachinon-2,3-disulfonsäure (I) durch Umsetzung eines 1,4-Diamino-2,3-dihalogenanthrachinons (II) mit Borsäure in einem inerten organischen Lösungsmittel und weitere Umsetzung des erhaltenen Reaktionsprodukts mit einer wäßrigen Sulfitlösung.

Weiterhin betrifft die Erfindung die Herstellung von 1,4-Diaminoanthrachinon-2,3-dinitril (III) durch Umsetzung der erfindungsgemäß erhaltenen 1,4-Diaminoanthrachinon-2,3-disulfonsäure (I) mit Cyanid.

1,4-Diaminoanthrachinon-2,3-disulfonsäure (I) und 1,4-Diaminoanthrachinon-2,3-dinitril (III) 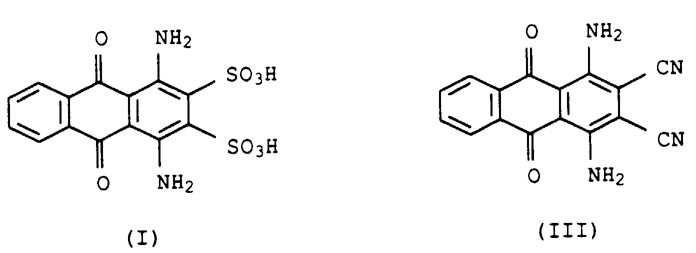 sind wertvolle Zwischenprodukte für die Herstellung von blauen Dispersionsfarbstoffen wie C.I. Disperse Blue 60.

Aus der US-A-4 279 825 ist die Herstellung von 1,4-Diaminoanthrachinon-2,3-disulfonsäure ausgehend von 1,4-Diamino-2,3-dichloranthrachinon bekannt. Dort wird das Dichloranthrachinon zunächst in Gegenwart von Nitrobenzol mit Borsäure umgesetzt, wobei das bei der Reaktion gebildete Wasser im Gemisch mit dem Nitrobenzol vollständig abdestilliert wird. Das als Zwischenstufe auftretende Reaktionsprodukt (IIa) aus Anthrachinon und Borsäure wird nach seiner Isolierung mit wäßrigem Sulfit zur Disulfonsäure umgesetzt.

Nachteilig bei diesem Verfahren ist, daß bei der Abdestillation von Nitrobenzol und Wasser gleichzeitig auch die im Überschuß eingesetzte, wasserdampfflüchtige Borsäure mitausgetrieben wird und zur Verstopfung an der jeweiligen Apparatur führen kann. Bei Verwendung des in der US-A-4 279 825 empfohlenen Schaufeltrockners zur Trocknung der Zwischenstufe (IIa) treten aufgrund der stark korrosiven Wirkung des Reaktionsgemisches weitere Probleme auf.

Die Disulfonsäure (II) kann dann, wie beispielsweise in der US-A-4 279 825 beschrieben, durch Umsetzung mit Cyanid in das Dinitril (III) überführt werden.

Der Erfindung lag die Aufgabe zugrunde, 1,4-Diaminoanthrachinon-2,3-disulfonsäure (I) ohne die genannten Nachteile auf verfahrenstechnisch einfache und wirtschaftliche Weise in guter Ausbeute und Reinheit herzustellen und damit auch eine vorteilhafte Herstellung von 1,4-Diaminoanthrachinon-2,3-dinitril (III) zu ermöglichen.

Demgemäß wurde ein Verfahren zur Herstellung von 1,4-Diaminoanthrachinon-2,3-disulfonsäure (I) durch Umsetzung eines 1,4-Diamino-2,3-dihalogenanthrachinons (II) mit Borsäure in einem inerten organischen Lösungsmittel und weitere Umsetzung des erhaltenen Reaktionsprodukts (IIa) mit einer wäßrigen Sulfitlösung gefunden, welches dadurch gekennzeichnet ist, daß man als inertes organisches Lösungsmittel ein unpolares Lösungsmittel mit einem Siedepunkt ≥ 130°C und einer Dichte ≤ 0,95 g/cm³ verwendet.

Die erfindungsgemäß hergestellte 1,4-Diaminoanthrachinon-2,3-disulfonsäure (I) kann dann durch Umsetzung mit Cyanid in das 1,4-Diaminoanthrachinon-2,3-dinitril (III) überführt werden.

Für die Umsetzung des 1,4-Diamino-2,3-dihalogenanthrachinons (II) mit Borsäure geeignete unpolare organische Lösungsmittel haben in der Regel einen Siedepunkt ≥ 130°C.

Dabei sind neben aliphatischen Kohlenwasserstoffen oder deren Gemischen, wie sie beispielsweise unter dem Namen Exsol® (Esso Chemie) im Handel sind, vor allem aromatische Kohlenwasserstoffe, insbesondere Alkylbenzole, die oberhalb 130°C sieden, bevorzugt. Als Beispiele für besonders bevorzugte Alkylbenzole seien m-, p- und o-Xylol sowie deren Mischungen, Ethylbenzol und Isopropylbenzol genannt. Besonders vorteilhaft ist die Verwendung ggeigneter Mischungen von Alkylbenzolen, die beispielsweise unter dem Namen Solvesso^{®} (Esso Chemie) erhältlich sind. Ganz besonders bevorzugt sind dabei Gemische mit einem Siedebereich von etwa 180 bis 200°C und einer Dichte von etwa 0,90 g/cm³ (Solvesso 150).

Eine obere Grenze für die Siedetemperatur des Lösungsmittels gibt es im Prinzip nicht, jedoch wird man aus praktischen Erwägungen die oberhalb etwa 250°C siedenden Kohlenwasserstoffe aufgrund ihrer Zähflüssigkeit nicht mehr als Reaktionsmedium verwenden. Ebenso könnten auch Alkylbenzole, die unterhalb von 130°C, insbesondere 140°C, sieden, eingesetzt werden, jedoch müßte dann die Reaktionstemperatur entsprechend erniedrigt werden, was eine unwirtschaftliche Erniedrigung der Reaktionsgeschwindigkeit zur Folge hätte. In der Regel werden also Lösungsmittel, die etwa bei 160 bis 220°C sieden, besonders bevorzugt sein.

Weiterhin sollte die Dichte des Lösungsmittels kleiner als diejenige einer wäßrigen Phase, insbesondere derjenigen bei der Sulfonierungsreaktion sein, d.h. die Dichte sollte im allgemeinen ≤ 0,95 g/cm³ betragen.

Der besondere Vorteil bei der Verwendung der erfindungsgemäßen Lösungsmittel liegt darin, daß diese nicht vor der Sulfonierungsreaktion abgetrennt werden müssen, sondern daß die Zwischenstufe (IIa) aus dem Anthrachinon (II) und Borsäure ohne Zwischenisolierung direkt in Form des anfallenden Reaktionsgemisches mit Sulfit zur Disulfonsäure (III) umgesetzt werden kann. Das Lösungsmittel kann dann anschließend in einfacher Weise als leichte Phase abgetrennt und erneut als Lösungsmittel eingesetzt werden. Die für das Verfahren der US-A-4 279 825 geschilderten Probleme treten beim erfindungsgemäßen Verfahren gar nicht erst auf, da auf die Isolierung der Zwischenstufe (IIa) verzichtet werden kann.

Die Menge an erfindungsgemäßem Lösungsmittel, insbesondere ALkylbenzol, ist an sich nicht kritisch; in der Regel werden 3 bis 5 kg pro kg (II) eingesetzt.

Die weiteren Verfahrensbedingungen können wie für diese Umsetzung üblich gewählt werden.

Bevorzugte Ausgangsverbindungen (II) sind sowohl 1,4-Diamino-2,3-dichlor- als auch -dibromanthrachinon.

Die Borsäure kann in Form der meta-Borsäure (HBO₂) oder in Form der bevorzugten ortho-Borsäure (H₃BO₃) eingesetzt werden.

Im allgemeinen beträgt die Menge Borsäure 2 bis 10 mol, bevorzugt 6 bis 8 mol pro mol II.

Verfahrenstechnisch geht man zweckmäßigerweise so vor, daß man das Gemisch aus 1,4-Diamino-2,3-dihalogenanthrachinon (II), Borsäure und erfindungsgemäßem Lösungsmittel in etwa 1 bis 3 h auf 120 bis 180°C, vorzugsweise 150 bis 165°C, gewünschtenfalls unter Inertgas, z.B. unter Durchleiten eines Stickstoffstroms, erhitzt. Dabei destilliert ein Teil des Reaktionswassers und des Lösungsmittels als Azeotrop ab. Nach einer Reaktionszeit von etwa 2 bis 3 h, in der sich das Reaktionsprodukt (IIa) aus Halogenanthrachinon (II) und Borsäure gebildet hat, kühlt man das Reaktionsgemisch auf in der Regel 90 bis 95°C ab und setzt es ohne weitere Behandlung zur Bildung der Disulfonsäure (I) ein.

Die Sulfonierungsreaktion selbst kann ebenfalls unter den üblichen Bedingungen durchgeführt werden.

Als wäßriges Sulfit kommen dabei vor allem Lösungen von Alkalisulfiten wie Kalium- und insbesondere Natriumsulfit, auch von Ammoniumsulfit in Betracht.

Im allgemeinen wird das Sulfit in einer Menge von etwa 2,2 bis 2,6 mol pro mol (II) als etwa 8 bis 10 gew.-%ige wäßrige Lösung eingesetzt.

Während der Zugabe des erhaltenen Reaktionsgemisches zu der auf 90 bis 95°C erhitzten Sulfitlösung wird der pH-Wert der Mischung zweckmäßigerweise durch gleichzeitige Zudosierung einer Base wie Natron- oder Kalilauge bei 7,5 bis 8,5 gehalten.

Diese Umsetzung dauert im allgemeinen etwa 30 bis 60 min.

Anschließend wird beim erfindungsgemäßen Verfahren, vorzugsweise nach einer Klärfiltration des Gesamtansatzes, das noch enthaltene unpolare Lösungsmittel als leichte Phase von der unteren, die Disulfonsäure (I) als Salz gelöst enthaltenden, wäßrigen Phase abgetrennt.

Auf eine Isolierung der 1,4-Diaminoanthrachinon-2,3-disulfonsäure (I) durch Ausfällen mit einer Säure, Abfiltrieren, Waschen und Trocknen kann verzichtet werden, vielmehr kann die abgetrennte, wäßrige Phase vorteilhaft direkt für Folgeumsetzungen in wäßrigem Medium, wie die Herstellung von 1,4-Diaminoanthrachinon-2,3-dinitril (III), verwendet werden.

Das erfindungsgemäße Verfahren zur Herstellung von 1,4-Diaminoanthrachinon-2,3-disulfonsäure (I) kann sowohl kontinuierlich als auch diskontinuierlich durchgeführt werden.

Mit seiner Hilfe wird die 1,4-Diaminoanthrachinon-2,3-disulfonsäure auf einfache, wirtschaftliche Weise in guter Ausbeute und hoher Reinheit erhalten, was sich nicht zuletzt auch in der Qualität und Ausbeute der Folgeprodukte zeigt.

Bei der weiteren Umsetzung zum 1,4-Diaminoanthrachinon-2,3-dinitril (III) kann man wie üblich und in der US-A-4 279 825 beschrieben vorgehen, indem man ein Alkalicyanid wie Kalium- und vor allem Natriumcyanid in Mengen von in der Regel 2,2 bis 4 mol pro mol (I) zugibt, das resultierende Gemisch für etwa 3 bis 5 h auf 90 bis 95°C erhitzt und überschüssiges Cyanid mit Hilfe von Wasserstoffperoxid vernichtet.

Auch das 1,4-Diaminoanthrachinon-2,3-dinitril (III) fällt in so hoher Reinheit an, daß es direkt für weitere Zwecke wie die Pigmentherstellung eingesetzt werden kann.

Die Herstellung des 1,4-Diaminoanthrachinon-2,3-dinitrils (III) über die erfindungsgemäße Sulfonierung eines 1,4-Diamino-2,3-dihalogenanthrachinons (II) und anschließende Umsetzung der so erhaltenen 1,4-Diaminoanthrachinon-2,3-disulfonsäure (I) mit Cyanid stellt einen vorteilhaften Syntheseweg dar.

### Beispiel 1

a) Herstellung von 1,4-Diaminoanthrachinon-2,3-disulfonsäure (I)
   Eine Mischung aus 400 g Solvesso 150, 103 g (0,26 mol) 1,4-Diamino-2,3-dibromanthrachinon (ber. 100 %) und 139 g (2,24 mol) ortho-Borsäure wurde unter schwachem Stickstoffstrom in 3 h auf 160-165°C erhitzt. Dabei destillierte langsam ein Wasser/Solvesso 150-Gemisch ab.
   Nach 2 h wurde die Suspension auf 90°C abgekühlt und zu einer 90°C heißen Lösung von 80 g Natriumsulfit in 1200 ml Wasser getropft. Durch gleichzeitiges Zutropfen von 50 gew.-%iger Natronlauge wurde dabei der pH-Wert der Lösung während der gesamten Eintragszeit zwischen 8 und 8,5 gehalten.
   Nach 30minütigem Rühren bei 90°C wurde der gesamte Reaktionsansatz klärfiltriert, und das Lösungsmittel wurde durch Phasentrennung abgetrennt. Die verbliebene wäßrige Lösung enthielt die 1,4-Diaminoanthrachinon-2,3-disulfonsäure.
b) Weiterreaktion zu 1,4-Diaminoanthrachinon-2,3-dinitril (III)
   Nach Zugabe von 36 g (0,73 mol) Natriumcyanid wurde die wäßrige Phase für 3 h auf 90 bis 95°C erhitzt. Nach Abkühlen auf 80°C wurden 120 ml 30 gew.-%iges Wasserstoffperoxid zur Vernichtung von überschüssigem Cyanid zugetropft.
   Der entstandene Niederschlag wurde heiß abfiltriert, mit heißem Wasser gewaschen und getrocknet.
   Es wurden 65,3 g 1,4-Diaminoanthrachinon-2,3-dinitril mit einem Reinheitsgrad von 91,4 % erhalten, was einer Ausbeute von 79,5 % entspricht.

### Beispiel 2

Analog Beispiel 1 wurden 80,0 g (0,26 mol) 1,4-Diamino-2,3-dichloranthrachinon (ber. 100 %) in 480 g Solvesso 150 umgesetzt.

Bei der Weiterreaktion der gebildeten 1,4-Diaminoanthrachinon-2,3-disulfonsäure wurden 64 g 1,4-Diaminoanthrachinon-2,3-dinitril mit einem Reinheitsgrad von 90 % erhalten, was einer Ausbeute von 77 % entspricht.

## Patentansprüche

1. Verfahren zur Herstellung von 1,4-Diaminoanthrachinon-2,3-disulfonsäure (I) durch Umsetzung eines 1,4-Diamino-2,3-dihalogenanthrachinons(II) mit Borsäure in einem inerten organischen Lösungsmittel und weitere Umsetzung des erhaltenen Reaktionsprodukts (IIa) mit einer wäßrigen Sulfitlösung, dadurch gekennzeichnet, daß man als inertes organisches Lösungsmittel ein unpolares Lösungsmittel mit einem Siedepunkt ≧ 130°C und einer Dichte ≦ 0,95 g/cm³ verwendet.

2. Verfahren nach Anspruch 1, dadurch gekennzeichnet, daß man als Lösungsmittel ein Alkylbenzol oder Alkylbenzolgemisch einsetzt.

3. Verfahren nach Anspruch 1 oder 2, dadurch gekennzeichnet, daß man das Reaktionsprodukt (IIa) ohne Zwischenisolierung in Form des anfallenden Reaktionsgemisches mit dem Sulfit umsetzt und das organische Lösungsmittel anschließend durch Phasentrennung entfernt.

4. Verfahren nach den Ansprüchen 1 bis 3, dadurch gekennzeichnet, daß man als 1,4-Diamino-2,3-dihalogenanthrachinon (II) 1,4-Diamino-2,3-dichlor- oder -dibromanthrachinon einsetzt.

5. Verfahren zur Herstellung von 1,4-Diaminoanthrachinon-2,3-dinitril (III) durch
a) Umsetzung eines 1,4-Diamino-2,3-dihalogenanthrachinons (II) mit Borsäure in einem inerten organischen Lösungsmittel und weitere Umsetzung des erhaltenen Reaktionsprodukts (IIa) mit einer wäßrigen Sulfitlösung und
b) anschließende Umsetzung der gebildeten 1,4-Diaminoanthrachinon-2,3-disulfonsäure(I) mit Cyanid,
dadurch gekennzeichnet, daß man als inertes organisches Lösungsmittel in Schritt a) ein unpolares Lösungsmittel mit einem Siedepunkt ≥ 130°C und eine Dichte ≤ 0,95 g/cm³ verwendet.

6. Verfahren nach Anspruch 5, dadurch gekennzeichnet, daß man als Lösungsmittel ein Alkylbenzol oder Alkylbenzolgemisch einsetzt.

## Claims

1. A process for preparing 1,4-diaminoanthraquinone-2,3-disulfonic acid (I) by reacting a 1,4-diamino-2,3-dihaloanthraquinone (II) with boric acid in an inert organic solvent and further reacting the resulting reaction product (IIa) with an aqueous sulfite solution, characterized in that the inert organic solvent used is an apolar solvent having a boiling point ≥ 130°C and a density ≤ 0.95 g/cm³.

2. A process as claimed in claim 1, characterized in that the solvent used is an alkylbenzene or an alkylbenzene mixture.

3. A process as claimed in claim 1 or 2, characterized in that the reaction product (IIa) is reacted with the sulfite without first being isolated in the form of the as-obtained reaction mixture and the organic solvent is subsequently removed by phase separation.

4. A process as claimed in any of claims 1 to 3, characterized in that the 1,4-diamino-2,3-dihaloanthraquinone (II) used is 1,4-diamino-2,3-dichloro- or -dibromo-anthraquinone.

5. A process for preparing 1,4-diaminoanthraquinone-2,3-dinitrile (III) by
a) reacting a 1,4-diamino-2,3-dihaloanthraquinone (II) with boric acid in an inert organic solvent and further reacting the resulting reaction product (III) with an aqueous sulfite solution, and
b) subsequently reacting the resulting 1,4-diaminoanthraquinone-2,3-disulfonic acid (I) with cyanide,
characterized in that the inert organic solvent used in step a) is an apolar solvent having a boiling point ≥ 130°C and a density ≤ 0.95 g/cm³.

6. A process as claimed in claim 5, characterized in that the solvent used is an alkylbenzene or an alkylbenzene mixture.

## Revendications

1. Procédé de préparation d'acide 1,4-diaminoanthraquinone-2,3-disulfonique (I) par réaction d'une 1,4-diamino-2,3-dihalogénoanthraquinone (II) avec de l'acide borique dans un solvant organique inerte et réaction ultérieure du produit de réaction (IIa) obtenu avec une solution aqueuse de sulfite, caractérisé en ce que l'on utilise, comme solvant organique inerte, un solvant non polaire ayant un point d'ébullition ≥ 130°C et une masse volumique ≤ 0,95 g/cm³.

2. Procédé selon la revendication 1, caractérisé en ce que l'on utilise, comme solvant, un alkylbenzène ou un mélange d'alkylbenzènes.

3. Procédé selon la revendication 1 ou 2, caractérisé en ce que l'on fait réagir le produit de réaction (IIa) avec le sulfite sans étape intermédiaire d'isolement, sous la forme du mélange réactionnel formé, puis on élimine le solvant organique par séparation de phases.

4. Procédé selon l'une quelconque des revendications 1 à 3, caractérisé en ce que l'on utilise, comme 1,4-diamino-2,3-dihalogénoanthraquinone (II), de la 1,4-diamino-2,3-dichloro- ou -dibromoanthraquinone.

5. Procédé de préparation de 1,4-diaminoanthraquinone-2,3-dinitrile (III) par
a) réaction d'une 1,4-diamino-2,3-dihalogénoanthraquinone (II) avec de l'acide borique dans un solvant organique inerte et réaction ultérieure du produit de réaction (IIa) obtenu avec une solution aqueuse de sulfite,
b) suivie de réaction de l'acide 1,4-diaminoanthraquinone-2,3-disulfonique (I) formé avec un cyanure,
caractérisé en ce que l'on utilise, comme solvant organique inerte dans l'étape a), un solvant homopolaire ayant un point d'ébullition ≥ 130°C et une masse volumique ≤ 0,95 g/cm³.

6. Procédé selon la revendication 5, caractérisé en ce que l'on utilise, comme solvant, un alkylbenzène ou un mélange d'alkylbenzènes.
